# FASCICULE DE BREVET EUROPEEN

(11) **EP 0 882 691 B1**
(45) Date de publication et mention de la délivrance du brevet: **14.05.2003**
(21) Numéro de dépôt: 98401202.1
(22) Date de dépôt: 19.05.1998
(51) Int. Cl.: C07B 61/00, B01J 19/24, C07C 2/30, C07C 6/04

(54) **Procédé et installation pour réaliser une réaction sur une charge organique, telle que la dimérisation ou la métathèse, en présence d'une phase polaire contenant un catalyseur**
Verfahren und Anlage zur Ausführung einer Reaktion, wie Dimerisierung oder Metathese, an organischen Einsätzen in Gegenwart einer Katalysator enthaltenden polaren Phase
Process and apparatus for carrying out a reaction, such as dimerisation or metathesis, on an organic feedstock in the presence of a polar phase containing a catalyst

(30) Priorité: 27.05.1997 FR 9706571
(43) Date de publication de la demande: 09.12.1998
(73) Titulaire: INSTITUT FRANCAIS DU PETROLE, 92852 Rueil-Malmaison Cedex (FR)
(72) Inventeur: Olivier, Hélène, 92500 Rueil Malmaison (FR); Commereuc, Dominique, 92190 Meudon (FR); Forestière, Alain, 69390 Vernaison (FR); Hugues, François, 69390 Vernaison (FR)

(56) Documents cités:
- EP-A- 0 448 445
- EP-A- 0 646 412
- FR-A- 2 611 700
- FR-A- 2 715 328
- GB-A- 2 017 144

## Description

L'objet de la présente invention est un procédé biphasique amélioré pour réaliser une réaction sur une charge organique (telle que la dimérisation, la co-dimérisation, l'oligomérisation ou la métathèse des oléfines) en continu, avec un catalyseur qui contient au moins un élément catalytique, dissous dans un milieu ionique non aqueux peu ou pas miscible avec les oléfines. En d'autre termes, il s'agit d'un procédé pour réaliser une réaction en catalyse biphasique. L'invention a également pour objet l'installation pour mettre en oeuvre ce procédé.

Comme décrit antérieurement un système à deux phases présente l'avantage d'une meilleure utilisation du catalyseur qu'une mise en oeuvre homogène à une phase. Cependant une mise en oeuvre en continu économique d'un système à deux phases pose un certain nombre de problèmes, tels que l'interférence des traces d'impuretés contenues dans les charges avec le catalyseur et la phase polaire. Ces impuretés sont l'eau, les alcools, les ethers, les composés azotés et soufrés. Elles réagissent sur le complexe catalyseur-phase polaire.

Le procédé, objet de l'invention, consiste à faire circuler à contre-courant la charge et la phase polaire dans au moins deux boucles de traitement. Il est ainsi obtenu un prétraitement de la charge dans la première boucle par la composition catalytique usée, c'est-à-dire ayant perdu la plus grande partie de son activité, cette composition catalytique usée, venant de la deuxième boucle, est alors retirée du procédé. Cette mise en oeuvre diminue la consommation du système catalyseur-phase polaire, évite une section pour l'élimination de l'élément de transition et permet un traitement hors site du catalyseur.

Le procédé selon l'invention est un procédé pour réaliser une réaction sur une charge organique, en présence d'une phase polaire contenant au moins une composition catalytique et qui résulte du mélange d'au moins un milieu non aqueux à caractère ionique pas ou peu miscible avec la phase organique, au moins un composé d'un élément catalytique et éventuellement au moins un cocatalyseur.

Selon le procédé, objet de l'invention, la charge à traiter et ledit milieu non-aqueux circulent à contre-courant entre au moins deux boucles de traitement, chaque boucle comportant au moins une zone de réaction reliée à au moins une zone de séparation des phases organique et polaire, la charge à traiter étant amenée au niveau de la zone de réaction de la première boucle et le milieu non-aqueux à caractère ionique frais, c'est-à-dire n'ayant pas encore servi, étant introduit au niveau de la zone de réaction de la seconde ou de la dernière boucle. Le ou les autres constituant(s) de la composition catalytique peuvent être introduit(s) à n'importe quel niveau dans le procédé. La phase polaire séparée dans la zone de séparation de la seconde boucle ou de chacune des boucles qui la suit, est envoyée vers la zone de réaction de la première boucle ou respectivement de la boucle qui la précède, alors que la phase organique séparée dans la zone de séparation de la première boucle, ou des boucles qui la suivent, est envoyée vers la zone de réaction de la seconde boucle ou respectivement de la boucle qui la suit. La phase organique obtenue à partir de la zone de séparation de la dernière boucle, et la phase polaire obtenue à partir de la zone de séparation de la première boucle sont soutirées du procédé.

Selon une variante préférée, une partie du milieu réactionnel d'une zone de réaction est soutirée à un niveau de ladite zone pour être réinjectée dans ladite zone.

Avantageusement, une partie au moins de la phase polaire soutirée d'une zone de séparation d'une boucle est recyclée dans la zone de réaction de la même boucle.

De préférence, ledit milieu non-aqueux à caractère ionique frais comprend également au moins une partie d'au moins un constituant de la composition catalytique. Dans ce cas-là, avantageusement le milieu non-aqueux à caractère ionique frais qui est introduit au niveau de la dernière boucle comprend également au moins un composé d'un élément de transition.

Il est également préféré un procédé dans lequel au moins une partie d'au moins un constituant de la composition catalytique est introduit au niveau de la zone de réaction de la première boucle. Dès lors, avantageusement du co-catalyseur frais est introduit dans la zone de réaction de la première boucle.

Selon un mode de réalisation de l'invention, il est introduit dans la zone de réaction de la dernière boucle une composition catalytique fraîche comprenant le milieu non-aqueux à caractère ionique frais, et au moins un composé d'un élément de transition et éventuellement au moins un co-catalyseur.

Dans un mode de réalisation à deux boucles illustré ci-après, le procédé comporte une première et une seconde boucle de traitement, chacune comportant une zone de réaction (A₁ et A₂ respectivement) reliée à une zone de séparation (B₁ et B₂ respectivement),
- la charge à traiter est amenée au niveau de la première zone de réaction A₁ ainsi que du co-catalyseur, le milieu non-aqueux à caractère ionique frais mélangé à au moins un composé d'un élément de transition et éventuellement à au moins une partie du co-catalyseur étant amené dans la seconde zone de réaction A₂,
- la phase polaire séparée de la zone de séparation B₂, est introduite dans la zone de réaction A₁, alors que la phase organique séparée dans la zone de séparation B₁ est introduite dans la zone de réaction A₂,
- la phase organique contenant les produits de réaction séparée dans la zone de séparation B₂, et la phase polaire usée séparée dans la zone de séparation B₁, étant soutirées du procédé.

Dans un mode de réalisation par exemple, une partie du milieu réactionnel est soutirée de la zone de réaction de la seconde boucle, refroidie et réinjectée dans la dite zone.

Le milieu non-aqueux à caractère ionique comprend au moins un sel dit « sel fondu » et les sels préférés selon l'invention ont pour formule générale Q⁺ A⁻ dans laquelle A⁻ représente un anion peu coordinant ou non coordinant. Sont préférés ceux susceptibles de former un sel liquide à basse température, c'est-à-dire en dessous de 150°C et avantageusement d'au plus 80°C, et de préférence en dessous de 50°C, par exemple les ions halogénoaluminates, les organohalogénoaluminates, les halogénogallates, les organoalogénogallates. Q⁺ représente un ammonium quaternaire et/ou un phosphonium quaternaire. Les ammonium et/ou phosphonium quaternaires répondent de préférence aux formules générales NR¹R²R³R⁴⁺ et PR¹R²R³R⁴⁺, ou aux formules générales R¹R²N=CR³R⁴⁺ et R¹R²P=CR³R⁴⁺ où R¹, R², R³ et R⁴, identiques ou différents, représentent l'hydrogène à l'exception du cation NH₄⁺ et de préférence un seul substituant peut représenter l'hydrogène, ou des restes hydrocarbyles ayant de 1 à 12 atomes de carbone, par exemple des groupements alkyles, saturés ou non saturés, cycloalkyls ou aromatiques, aryl ou aralkyl, comprenant de 1 à 12 atomes de carbone. Les ammonium et/ou phosphonium peuvent également être dérivés d'hétérocycles azotés ou phosphorés comportant 1, 2 ou 3 atomes d'azote et/ou de phosphore, de formule générales: 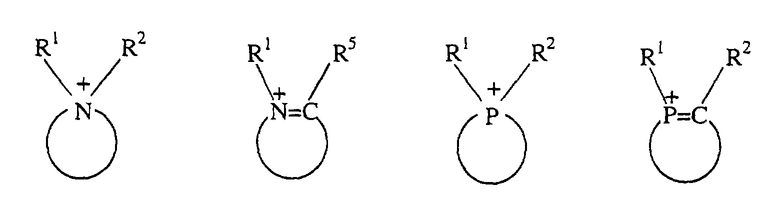 dans lesquelles les cycles sont consitués de 4 à 10 atomes, de préférence 5 à 6 atomes, R¹ et R² étant définis comme précédemment. L'ammonium ou le phosphonium quaternaire peuvent également être un cation de formule:

R¹R²⁺N=CR³-R⁵-R³C=N⁺R¹R²

R¹R²⁺P=CR³-R⁵-R³C=P⁺R¹R²

dans laquelle R¹,R²,R³, identiques ou différents sont définis comme précédemment et R⁵ représente un reste alkylène ou phénylène. Parmi les groupements R¹, R², R³ et R⁴ on mentionnera les radicaux méthyl, éthyl, propyl, isopropyl, butyl, secondaire butyl, tertiaire butyl, amyl, méthylène, éthylidène, phényl ou benzyl; R⁵ pourra être un groupement méthylène, éthylène, propylène ou phénylène. Le cation ammonium et/ou phosphonium est choisi de préférence dans le groupe formé par le N-butylpyridinium, le N-éthylpyridinium, le butyl-3 méthyl-1 imidazolium, le diéthylpyrazolium, l'éthyl-3 méthyl-1 imidazolium, le pyridinium, le triméthylphénylammonium, l'éthyl-3 méthyl-1 imidazolium, le tétrabutylphosphonium.
Ces sels peuvent être utilisés seuls ou en mélange. Ils ont une fonction de solvant.

Pour la dimérisation, co-dimérisation, oligomérisation, par exemple la phase polaire selon l'invention pourra aussi comprendre d'un mélange d'au moins un halogénure de lithium avec au moins un halogénure d'hydrocarbylaluminium (ainsi que le décrit dans la demande de brevet EP-A-753.346).

Dans un autre mode de réalisation, elle pourra comprendre un mélange d'au moins un halogénure d'ammonium ou de phosphonium quaternaire avec au moins un halogénure d'aluminium et/ou au moins un dihalogénure d'hydrocarbylaluminium (ainsi que le décrit dans. la demande de brevet EP-A-448.445 et FR-A2.611.700 et EP-A-646.412). L'élément catalytique préféré est le nickel.

La phase polaire préférée selon l'invention comprend (ou avantageusement est constituée par) le mélange d'au moins un halogénure d'aluminium ou respectivement d'au moins un halogénure d'hydrocarbyl aluminium avec au moins un halogénure d'ammonium quaternaire et/ou de phosphonium quaternaire, ou respectivement de lithium, et avantageusement dans un rapport molaire composé d'aluminium/sel fondu compris entre 1 et 2, et de préférence 1,1 à 1,6.

L'halogénure d'hydrocarbylaluminium a pour formule générale Al₂XₓR₆₋ₓ dans laquelle X est le chlore ou le brome, R est un reste alkyl, cycloalkyl, aryl ou aralkyl comportant de 1 à 10 et de préférence 2 à 6 atomes de carbone, x prenant les valeurs de 2, 3 ou 4. Ils peuvent être utilisés seuls ou en mélange. A titre d'exemple, on peut citer les chlorures d'alkylaluminium tels que le dichlorure d'éthylaluminium, le dichlorure d'isobutylaluminium, le sesquichlorure d'éthylaluminium, le chlorure de diéthylaluminium.

L'élément catalytique (généralement un métal de transition des groupes 6, 8, 9 ou 10) est mis en oeuvre sous la forme d'un sel tel que carboxylate, acétylacétonate, chlorure, bromure, sulfate, nitrate, ou les complexes que font ces sels avec les phosphines, amines, nitriles. D'autres composés peuvent convenir.

La concentration du composé du métal de transition dans la phase polaire est avantageusement comprise entre 1 mmole par litre et 500 mmoles par litre, de préférence entre 2 et 300 mmoles par litre.

L'invention n'est pas limitée aux compositions catalytiques ci-dessus précisées, d'autres compostions peuvent convenir, par la dimérisation, la co-dimérisation ou l'oligomérisation.
Pour la métathèse, on pourra par exemple travailler avec une composition décrite dans la demande de brevet FR-2.715.328, comprenant au moins un halogénure d'ammonium et/ou de phosphonium quaternaire mélangé à au moins un halogénure d'aluminium et au moins un composé organométallique de l'aluminium. Ce dernier a généralement pour formule Al₂XₓR₃₋ₓ où R est un radical alkyl, linéaire ou ramifié, comportant de 2 à 8 atomes de carbone, X est le chlore ou le brome et x est égal à 1, 2 ou 3. L'élément catalytique est le tungstène ou le molybdène (groupe 6).
D'autres catalyseurs peuvent convenir.
De façon plus générale, on choisira le catalyseur convenant pour réaliser la réaction recherchée.

Le mélange réactionnel comprend une phase hydrocarbonée constituée des réactifs, des produits et des constituants inertes, et de la phase polaire contenant le complexe du métal de transition dissous.

Les températures auxquelles se font la réaction et la séparation sont généralement sensiblement identiques et sont comprises généralement entre -20 et + 80°C, de préférence -10 à +60°C, la pression étant suffisante pour maintenir la totalité des réactifs et des constituants des coupes en phase liquide, c'est-à-dire en absence de toute phase gazeuse. Les conditions opératoires sont évidemment celles nécessaires pour réaliser la réaction recherchée.

L'invention a également pour objet une installation pour la mise en oeuvre d'une réaction dans un milieu non-aqueux à caractère ionique et peu ou pas miscible avec les oléfines, et comportant:
- au moins deux zones de réaction A₁ et A₂;
- au moins deux zones de séparation B₁ et B₂ pour séparer la phase hydrocarbonée de la phase polaire, lesdites zones étant reliées aux zones de réaction A₁ et A₂;
- au moins une conduite 1 pour l'introduction de la phase polaire dans la zone de réaction A₂;
- au moins une conduite 2 pour l'introduction de la charge d'oléfines dans la zone de réaction A₁;
- au moins une conduite 3 pour amener la phase hydrocarbonée séparée dans la zone de séparation B₁ vers la zone de réaction A₂;
- au moins une conduite 4 pour soutirer la phase hydrocarbonée produite séparée dans la zone de séparation B₂;
- au moins une conduite 5 pour envoyer vers la zone de réaction A₁ la phase polaire séparée dans la zone de séparation B₂;
- au moins une conduite 6 permettant le soutirage de la phase polaire usée séparée dans la zone de séparation B₁.

De préférence, l'installation peut comporter en outre les moyens suivants pris seul ou en combinaison :
- au moins une conduite 7 reliant la zone de séparation B₂ à la zone de réaction A₂ pour recycler la phase polaire.
- au moins une conduite 8 pour soutirer une partie du milieu réactionnel de la zone A₂ et la réintroduire dans ladite zone.
- au moins une conduite 9 pour soutirer une partie du milieu réactionnel de la zone A₁ et la réintroduire dans ladite zone.
- au moins une conduite 10 reliant la zone de séparation B₁ à la zone A₁ pour recycler la phase polaire.
- au moins une conduite 11 pour introduire les réactifs dans l'installation.

Avantageusement, les conduites 8 et 9 comportent des échangeurs de chaleur.

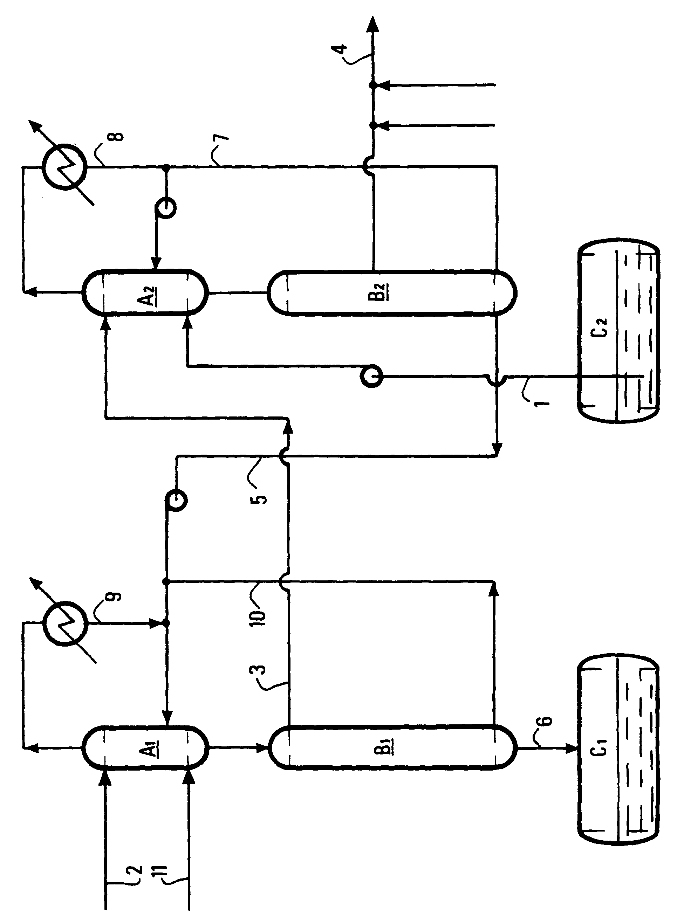

On comprendra mieux le procédé et l'installation à partir de la description de la figure 1. La figure 1 illustre un mode de réalisation d'une installation à deux boucles, également objet de l'invention, pour mettre en oeuvre le procédé.

Le système réactionnel, selon la figure, comprend deux boucles de traitement référencées I et II constituées des zones de réaction A1 et A2 et des zones de séparation B1 et B2. Les zones de réaction A1 et A2 sont constituées, par exemple, d'un réacteur muni d'un système d'agitation mécanique et refroidi extérieurement et/ou intérieurement, ou d'un réacteur muni d'une recirculation extérieure avec un échangeur de chaleur ou encore d'un réacteur tubulaire faisant office d'échangeur de chaleur. Les zones de séparation B1 et B2 sont constituées, par exemple, d'un récipient vertical dont la hauteur est suffisante pour assurer une bonne décantation de la phase polaire. Un système de régulation permet d'assurer un niveau constant de la phase polaire dans le séparateur. Dans la boucle I entre la charge oléfinique et de cette même boucle sort la phase polaire catalytique usée. Dans la boucle II entre la phase polaire catalytique fraîche et de cette même boucle sort la phase organique.

Ainsi, on introduit en continu, par l'intermédiaire de la conduite 2, dans la zone réactionnelle A1 la ou les oléfines pures ou en mélange avec des hydrocarbures saturés tels qu'ils sont obtenus dans les procédés de raffinage. On y introduit également, par l'intermédiaire de la conduite 11, la totalité ou une partie d'un constituant du catalyseur, tel qu'un composé organohalogéné de l'aluminium pur ou dilué par un hydrocarbure (dans le cas d'une réaction de dimérisation par exemple). La phase polaire "usée" contenue dans le séparateur B1 est envoyée dans le réservoir C1 par l'intermédiaire de la conduite 6 à un débit tel que son niveau dans le séparateur B1 reste stable. La phase hydrocarbonée contenue dans le séparateur B1 passe par l'intermédiaire de la conduite 3 dans la zone réactionnelle A2 au débit pondéral de la charge (boucles pleines, sans phase gazeuse). On introduit dans la zone réactionnelle A2, par l'intermédiaire de la conduite 1, la phase polaire "fraîche" contenant l'élément catalytique, le sel de nickel (pour une réaction de dimérisation, par exemple) et contenue dans le réservoir C2.

La phase polaire contenue dans le séparateur B2 est reprise par une pompe et envoyée par l'intermédiaire de la conduite 5 dans la zone réactionnelle Al à un débit tel que le niveau de la phase polaire dans le séparateur B2 reste constant. Une partie est renvoyée au réacteur A2. La phase hydrocarbonée contenue dans le séparateur B2 est soutirée par l'intermédiaire de la conduite 4 au débit pondéral de la charge (boucles pleines, sans phase gazeuse). Elle contient de petites quantités du composé organohalogéné de l'aluminium et est traitée par de l'ammoniaque anhydre puis lavée par une solution aqueuse de soude puis par de l'eau. Elle est ensuite soumise à un fractionnement qui permet de séparer les hydrocarbures qui n'ont pas réagi, les dimères et co-dimères et les oligomères supérieurs. Les réacteurs A1 et A2 sont munis de conduites de recirculation externe, respectivement 9 et 8, pour assurer le mélange des deux phases et éliminer la chaleur de réaction au travers d'échangeurs.

L'exemple suivant, qui décrit la dimérisation des n-butènes contenus dans une coupe C₄ issue de craquage à la vapeur d'un naphta, au moyen d'un composé du nickel et de dichloroéthyl aluminium, dissous dans une phase polaire constituée de chlorure de butyl-1 méthyl-3 imidazolium et de chlorure d'aluminium, illustre l'invention sans en limiter la portée.

### Exemple

Les deux boucles sont constituées chacune d'un réacteur et d'un échangeur dont le volume total est de 500 litres ainsi que d'un séparateur. Le séparateur est un réservoir cylindrique de 5 mètres de hauteur et de 0,5 mètre de diamètre.
Dans le réacteur de la première boucle on introduit au débit de 3900 kg/heure une coupe C₄ constituée de 70% de n-butènes et de 2% d'isobutène le reste étant constitué d'alcanes. On y introduit également, au débit de 9,6 kg/h, une solution de dichloroéthylaluminium à 50% en poids dans de l'hexane.
Dans le réacteur de la deuxième boucle on introduit au débit de 1,25 kg/heure une phase polaire contenue dans le réservoir C2 et constituée de 0,577 kg de chlorure d'aluminium, 0,622 kg de chlorure de butylméthylimidazolium et de 50 g de chlorure de nickel anhydre (rapport molaire chlorure d'aluminium: chlorure d'imidazolium =1,22). On maintient la température des zones de réaction et de séparation vers 10°C.
La phase polaire est reprise du séparateur B2 et envoyée dans la zone réactionnelle A1. La phase polaire qui s'accumule dans le séparateur B1 est envoyée dans le réservoir C1.
Au sortir du séparateur B2 l'effluent passant au travers de la conduite 4 était traité par de l'ammoniaque anhydre, puis par une solution aqueuse de soude à 20% et enfin par de l'eau. La conversion des n-butènes était de 80%; les produits étaient constitués à 95% de dimères et à 5% de trimères.

Comme le montre l'exemple, l'invention est particulièrement bien adaptée pour la transformation en continu des oléfines, que se soit en dimérisation, co-dimérisation, oligomérisation, comme illustré, ou encore par exemple en métathèse. Les catalyseurs étant bien entendu ceux adaptés pour réaliser la réaction.

## Revendications

1. Procédé pour réaliser une réaction sur une charge organique, en présence d'une phase polaire contenant au moins une composition catalytique et qui résulte du mélange
1) d'au moins un milieu non-aqueux à caractère ionique pas ou peu miscible avec la phase organique,
2) au moins un composé d'un élément catalytique,
3) et éventuellement au moins un co-catalyseur,
dans lequel la charge à traiter et ledit milieu non-aqueux circulent à contre-courant entre au moins deux boucles de traitement, chaque boucle comportant au moins une zone de réaction reliée à au moins une zone de séparation des phases organique et polaire, la charge à traiter étant amenée au niveau de la zone de réaction de la première boucle et le milieu non-aqueux à caractère ionique frais étant introduit au niveau de la zone de réaction de la seconde ou de la dernière boucle, le ou les autres constituant(s) de la composition catalytique étant introduit(s) à n'importe quel niveau dans le procédé, et la phase polaire séparée dans la zone de séparation de la seconde boucle de chacune des boucles qui la suit, est envoyée vers la zone de réaction de la première boucle ou respectivement de la boucle qui la précède, alors que la phase organique séparée dans la zone de séparation de la première boucle, ou des boucles qui la suivent, est envoyée vers la zone de réaction de la seconde boucle ou respectivement de la boucle qui la suit, la phase organique produite obtenue à partir de la zone de séparation de la dernière boucle, et la phase polaire obtenue à partir de la zone de séparation de la première boucle étant soutirées du procédé.

2. Procédé selon la revendication 1, dans lequel une partie du milieu réactionnel d'une zone de réaction est soutirée à un niveau de ladite zone pour être réinjectée dans ladite zone.

3. Procédé selon l'une des revendications précédentes dans lequel une partie au moins de la phase polaire soutirée d'une zone de séparation d'une boucle est recyclée dans la zone de réaction de la même boucle.

4. Procédé selon l'une des revendications précédentes, dans lequel ledit milieu non-aqueux à caractère ionique frais comprend également au moins une partie d'au moins un constituant de la composition catalytique.

5. Procédé selon la revendication 4 dans lequel le milieu non-aqueux à caractère ionique frais qui est introduit au niveau de la dernière boucle comprend également au moins un composé d'un élément de transition.

6. Procédé selon l'une des revendications précédentes, dans lequel au moins une partie d'au moins un constituant de la composition catalytique est introduit au niveau de la zone de réaction de la première boucle.

7. Procédé selon la revendication 6, dans lequel du co-catalyseur frais est introduit dans la zone de réaction de la première boucle.

8. Procédé selon l'une des revendications précédentes, dans lequel il est introduit dans la zone de réaction de la dernière boucle une composition catalytique fraîche comprenant le milieu non-aqueux à caractère ionique frais, et au moins un composé d'un élément de transition et éventuellement au moins un co-catalyseur.

9. Procédé selon l'une des revendications précédentes, comportant une première et une seconde boucle de traitement, chacune comportant une zone de réaction (A₁ et A₂ respectivement) reliée à une zone de séparation (B₁ et B₂ respectivement),
- la charge à traiter est amenée au niveau de la première zone de réaction A₁ ainsi que du co-catalyseur, le milieu non-aqueux à caractère ionique frais mélangé à au moins un composé d'un élément de transition et éventuellement à au moins une partie du co-catalyseur étant amené dans la seconde zone de réaction A₂,
- la phase polaire séparée de la zone de séparation B₂, est introduite dans la zone de réaction A₁, alors que la phase organique séparée dans la zone de séparation B₁ est introduite dans la zone de réaction A₂,
- la phase organique contenant les produits de réaction séparée dans la zone de séparation B₂, et la phase polaire usée séparée dans la zone de séparation B₁, étant soutirées du procédé.

10. Procédé selon les revendications 2 ou 9, dans lequel une partie du milieu réactionnel est soutirée de la zone de réaction de la seconde boucle, refroidie et réinjectée dans la dite zone.

11. Procédé selon l'une des revendications précédentes, dans lequel le milieu non-aqueux à caractère ionique comprend au moins un sel de formule Q⁺A⁻ où Q⁺ est un cation ammonium quaternaire ou phosphonium quaternaire et A⁻ est un anion coordinant ou non-coordinant.

12. Procédé selon la revendication 11, dans lequel l'anion est choisi dans le groupe formé par les halogénoaluminates, les organohalogénoaluminates, les halogénogallates, les organohalogénogallates.

13. Procédé selon l'une des revendications 1 à 10, dans lequel la phase polaire comprend un mélange d'au moins un halogénure d'ammonium quaternaire ou de phosphonium quaternaire avec au moins un halogénure d'aluminium et/ou au moins un dihalogénure d'hydrocarbylaluminium.

14. Procédé selon l'une des revendications 1 à 10, dans lequel le milieu non-aqueux à caractère ionique résulte du mélange d'au moins un halogénure de lithium avec au moins un halogénure d'hydrocarbylaluminium.

15. Procédé selon l'une des revendications précédentes, dans lequel la phase polaire comprend par le mélange d'au moins un halogénure d'aluminium ou respectivement d'au moins un halogénure d'hydrocarbyl aluminium avec au moins un halogénure d'ammonium quaternaire et/ou de phosphonium quaternaire, ou respectivement de lithium, dans un rapport molaire compris entre 1 et 2.

16. Procédé selon l'une des revendications précédentes, dans lequel la composition catalytique contient au moins un élément catalytique choisi dans le groupe formé par les éléments des groupes 6, 8, 9 et 10.

17. Procédé selon l'une des revendications précédentes, utilisé pour la réaction de dimérisation, co-dimérisation ou oligomérisation d'oléfines.

18. Procédé selon la revendication 17, dans lequel la composition catalytique comprend au moins un composé du nickel, au moins un halogénure d'ammonium quaternaire et/ou de phosphonium quaternaire, et au moins un halogénure d'aluminium et/ou au moins un dihalogénure d'hydrocarbylaluminium.

19. Procédé selon l'une des revendications 1 à 16, utilisé pour la réaction de métathèse des oléfines.

20. Procédé selon la revendication 19, dans lequel l'élément catalytique est le tungstène et/ou le molybdène, le co-catalyseur est un composé organométallique de l'aluminium, et le milieu non-aqueux à caractère ionique résulte du mélange d'au moins
un halogénure d'ammonium et/ou de phosphonium quaternaire, et au moins un halogénure d'aluminium et/ou au moins un dihalogénure d'hydrocarbylaluminium.

21. Installation pour la mise en oeuvre d'une réaction dans un milieu non-aqueux à caractère ionique et peu ou pas miscible avec les oléfines, et comportant:
- au moins deux zones de réaction A₁ et A2;
- au moins deux zones de séparation B₁ et B₂ pour séparer la phase hydrocarbonée de la phase polaire, lesdites zones étant reliées aux zones de réaction A₁ et A₂;
- au moins une conduite 1 pour l'introduction de la phase polaire dans la zone de réaction A₂;
- au moins une conduite 2 pour l'introduction de la charge d'oléfines dans la zone de réaction A₁;
- au moins une conduite 3 pour amener la phase hydrocarbonée séparée dans la zone de séparation B₁ vers la zone de réaction A₂;
- au moins une conduite 4 pour soutirer la phase hydrocarbonée produite séparée dans la zone de séparation B₂;
- au moins une conduite 5 pour envoyer vers la zone de réaction A₁ la phase polaire séparée dans la zone de séparation B₂;
- au moins une conduite 6 permettant le soutirage de la phase polaire usée séparée dans la zone de séparation B₁.

22. Installation selon la revendication 21, comportant en outre au moins une conduite 7 reliant la zone de séparation B₂ à la zone de réaction A₂ pour recycler la phase polaire.

23. Installation selon l'une des revendications 21 et 22, comportant en outre au moins une conduite 8 pour soutirer une partie du milieu réactionnel de la zone A₂ et la réintroduire dans ladite zone.

24. Installation selon l'une des revendications 21 à 23, comportant en outre au moins une conduite 9 pour soutirer une partie du milieu réactionnel de la zone A₁ et la réintroduire dans ladite zone.

25. Installation selon l'une des revendications 21 à 24, comportant en outre au moins une conduite 10 reliant la zone de séparation B₁ à la zone A₁ pour recycler la phase polaire.

26. Installation selon l'une des revendication 21 à 25, comportant en outre au moins une conduite 11 pour introduire les réactifs dans ladite installation.

27. Installation selon l'une des revendications 21 à 26, dans laquelle les conduites 8 et 9 comportent des échangeurs de chaleur.

28. Utilisation d'une installation selon les revendications 21 à 27 pour la réaction de dimérisation, co-dimérisation ou oligomérisation d'oléfines.

29. Utilisation d'une installation selon les revendications 21 à 27 pour la réaction de métathèse des oléfines.

## Patentansprüche

1. Verfahren zum Durchführen einer Reaktion mit einer organischen Beschickung in Anwesenheit einer polaren Phase, die zumindest eine katalytische Zusammensetzung enthält und die resultiert aus dem Gemisch
1) aus zumindest einem nicht-wässrigen ionischen Medium, das nicht oder kaum mit der organischen Phase mischbar ist,
2) zumindest einer Verbindung eines katalytischen Elementes,
3) und eventuell zumindest eines Cokatalysators,
in dem die zu verarbeitende Beschickung und das nicht-wässrige Medium im Gegenstrom zwischen zumindest zwei Verarbeitungsschleifen zirkulieren, wobei jede Schleife zumindest eine Reaktionszone umfasst, die mit zumindest einer Zone zur Trennung der organischen und polaren Phasen verbunden ist, die zu verarbeitende Beschickung zur Reaktionszone der ersten Schleife verbracht wird, und das frische nicht-wässrige ionische Medium bei der Reaktionszone der zweiten oder der letzten Schleife eingespeist wird, wobei der oder die anderen Bestandteil(e) der katalytischen Zusammensetzung an egal welcher Stelle im Verfahren eingespeist wird (werden), und wobei die in der Zone zur Trennung der zweiten Schleife jeder der Schleifen, die ihr folgt, abgetrennte polare Phase zur Reaktionszone der ersten Schleife, beziehungsweise der Schleife, die ihr vorangeht, verbracht wird, während die in der Zone zur Trennung der ersten Schleife, oder der Schleifen, die ihr folgen, abgetrennte organische Phase zur Reaktionszone der zweiten Schleife, beziehungsweise der Schleife, die ihr folgt, verbracht wird, und wobei die aus der Zone zur Trennung der letzten Schleife erhaltene hergestellte organische Phase, und die aus der Zone zur Trennung der ersten Schleife erhaltene polare Phase aus dem Verfahren abgezapft werden.

2. Verfahren gemäß Anspruch 1, in dem ein Teil des Reaktionsmediums einer Reaktionszone aus einem Teil der Zone abgezapft wird, um wieder in die Zone eingespeist zu werden.

3. Verfahren gemäß einem der vorhergehenden Ansprüche, in dem zumindest ein Teil der aus einer Zone zur Trennung einer Schleife abgezapften polaren Phase in die Reaktionszone derselben Schleife zurückgeführt wird.

4. Verfahren gemäß einem der vorhergehenden Ansprüche, in dem das frische nicht-wässrige ionische Medium auch zumindest einen Teil zumindest eines Bestandteils der katalytischen Zusammensetzung enthält.

5. Verfahren gemäß Anspruch 4, in dem das frische nicht-wässrige ionische Medium, das bei der letzten Schleife eingespeist wird, auch zumindest eine Verbindung eines Übergangselementes enthält.

6. Verfahren gemäß einem der vorhergehenden Ansprüche, in dem zumindest ein Teil zumindest eines Bestandteils der katalytischen Zusammensetzung bei der Reaktionszone der ersten Schleife eingespeist wird.

7. Verfahren gemäß Anspruch 6, in dem frischer Cokatalysator in die Reaktionszone der ersten Schleife eingespeist wird.

8. Verfahren gemäß einem der vorhergehenden Ansprüche, in dem in die Reaktionszone der letzten Schleife eine frische katalytische Zusammensetzung, umfassend das frische nicht-wässrige ionische Medium, und zumindest eine Verbindung eines Übergangselementes und eventuell zumindest einen Cokatalysator, eingespeist wird.

9. Verfahren gemäß einem der vorhergehenden Ansprüche, umfassend eine erste und eine zweite Verarbeitungsschleife, jede umfassend eine mit einer Zone zur Trennung (jeweils B₁ und B₂) verbundene Reaktionszone (jeweils A₁ und A₂),
- die zu verarbeitende Beschickung wird, ebenso wie Cokatalysator, zur ersten Reaktionszone A₁ verbracht, wobei das frische nicht-wässrige ionische Medium, vermischt mit zumindest einer Verbindung eines Übergangselementes und eventuell mit zumindest einem Teil des Cokatalysators, in die zweite Reaktionszone A₂ verbracht wird,
- die aus der Zone zur Trennung B₂ abgetrennte polare Phase wird in die Reaktionszone A₁ verbracht, während die aus der Zone zur Trennung B₁ abgetrennte organische Phase in die Reaktionszone A₂ verbracht wird,
- wobei die organische Phase, enthaltend die in der Zone zur Trennung B₂ abgetrennten Reaktionsprodukte, und die in der Zone zur Trennung B₁ abgetrennte verbrauchte polare Phase aus dem Verfahren abgezapft werden.

10. Verfahren gemäß Anspruch 2 oder 9, in dem ein Teil des Reaktionsmediums aus der Reaktionszone der zweiten Schleife abgezapft, gekühlt und wieder in die Zone eingespeist wird.

11. Verfahren gemäß einem der vorhergehenden Ansprüche, in dem das nicht-wässrige ionische Medium zumindest ein Salz der Formel Q⁺A⁻ enthält, worin Q⁺ ein quatemäres Ammonium- oder quaternäres Phosphoniumkation ist, und A⁻ ein koordinierendes oder nicht koordinierendes Anion ist.

12. Verfahren gemäß Anspruch 11, in dem das Anion gewählt ist aus der aus den Halogenaluminaten, den Organohalogenaluminaten, den Halogengallaten, den Organohalogengallaten gebildeten Gruppe.

13. Verfahren gemäß einem der Ansprüche 1 bis 10, in dem die polare Phase ein Gemisch zumindest eines quaternären Ammonium- oder quatemären Phosphoniumhalogenids mit zumindest einem Aluminiumhalogenid und/oder zumindest einem Hydrocarbylaluminiumdihalogenid enthält.

14. Verfahren gemäß einem der Ansprüche 1 bis 10, in dem das nicht-wässrige ionische Medium aus dem Gemisch aus zumindest einem Lithiumhalogenid mit zumindest einem Hydrocarbylaluminiumhalogenid resultiert.

15. Verfahren gemäß einem der vorhergehenden Ansprüche, in dem die polare Phase ein Gemisch von zumindest einem Aluminiumhalogenid, beziehungsweise zumindest einem Hydrocarbylaluminiumhalogenid mit zumindest einem quaternären Ammonium- und/oder quatemären Phosphonium-, beziehungsweise Lithiumhalogenid in einem molaren Verhältnis im Bereich zwischen 1 und 2 umfasst.

16. Verfahren gemäß einem der vorhergehenden Ansprüche, in dem die katalytische Zusammensetzung zumindest ein katalytisches Element enthält, ausgewählt aus der durch die Elemente der Gruppen 6, 8, 9 und 10 gebildeten Gruppe.

17. Verfahren gemäß einem der vorhergehenden Ansprüche, das zur Dimerisierung, Codimerisierung oder Oligomerisierung von Olefinen verwendet wird.

18. Verfahren gemäß Anspruch 17, in dem die katalytische Zusammensetzung zumindest eine Verbindung des Nickels, zumindest ein quaternäres Ammoniumund/oder quatemäres Phosphoniumhalogenid, und zumindest ein Aluminiumhalogenid und/oder zumindest ein Hydroalkylaluminiumdihalogenid umfasst.

19. Verfahren gemäß einem der Ansprüche 1 bis 16, das zur Olefinmetathese verwendet wird.

20. Verfahren gemäß Anspruch 19, in dem das katalytische Element Wolfram und/oder Molybdän ist, der Cokatalysator eine organometallische Verbindung des Aluminiums ist, und das nicht-wässrige ionische Medium resultiert aus der Mischung aus zumindest
einem Ammonium- und/oder quaternären Phosphoniumhalogenid, und zumindest einem Aluminiumhalogenid und/oder zumindest einem Hydrocarbylaluminiumdihalogenid.

21. Vorrichtung zur Durchführung einer Reaktion in einem nicht-wässrigen ionischen Medium, das nicht oder kaum mischbar mit Olefinen ist, umfassend:
- zumindest zwei Reaktionszonen A₁ und A₂;
- zumindest zwei Zonen zur Trennung B₁ und B₂ zum Trennen der Kohlenwasserstoffphase von der polaren Phase, wobei die Zonen mit den Reaktionszonen A₁ und A₂ verbunden sind;
- zumindest eine Leitung 1 zur Einspeisung der polaren Phase in die Reaktionszone A₂;
- zumindest eine Leitung 2 zur Einspeisung der Olefin-Beschickung in die Reaktionszone A₁;
- zumindest eine Leitung 3 zum Verbringen der in der Zone zur Trennung B₂ abgetrennten Kohlenwasserstoffphase zu der Reaktionszone A₂;
- zumindest eine Leitung 4 zum Abzapfen der hergestellten, in der Zone zur Trennung B₂ abgetrennten Kohlenwasserstoffphase;
- zumindest eine Leitung 5 zum Verbringen der in der Zone zur Trennung B₂ abgetrennten polaren Phase zu der Reaktionszone A₂;
- zumindest eine Leitung 6, die das Abzapfen der verbrauchten, in der Zone zur Trennung B₂ abgetrennten polaren Phase gestattet.

22. Vorrichtung gemäß Anspruch 21, außerdem umfassend zumindest eine die Zone zur Trennung B₂ mit der Reaktionszone A₂ verbindende Leitung 7 zum Zurückführen der polaren Phase.

23. Vorrichtung gemäß einem der Ansprüche 21 und 22, außerdem umfassend zumindest eine Leitung 8 zum Abzapfen eines Teils des Reaktionsmediums aus der Zone A₂ und Wiedereinspeisen dessen in die Zone.

24. Vorrichtung gemäß einem der Ansprüche 21 bis 23, außerdem umfassend zumindest eine Leitung 9 zum Abzapfen eines Teils des Reaktionsmediums aus der Zone A₁ und Wiedereinspeisen dessen in die Zone.

25. Vorrichtung gemäß einem der Ansprüche 21 bis 24, außerdem umfassend zumindest eine die Zone zur Trennung B₁ mit der Zone A₁ verbindende Leitung 10 zum Zurückführen der polaren Phase.

26. Vorrichtung gemäß einem der Ansprüche 21 bis 25, außerdem umfassend zumindest eine Leitung 11 zum Einspeisen der Reaktanden in die Vorrichtung.

27. Verwendung einer Vorrichtung gemäß einem der Ansprüche 21 bis 26, in der die Leitungen 8 und 9 Wärmeaustauscher umfassen.

28. Verwendung einer Vorrichtung gemäß den Ansprüchen 21 bis 27 zur Dimerisierung, Codimerisierung oder Oligomerisierung von Olefinen.

29. Verwendung einer Vorrichtung gemäß den Ansprüchen 21 bis 27 zur Olefinmetathese.

## Claims

1. A process for carrying out a reaction on an organic feed, in the presence of a polar phase containing at least one catalytic composition resulting from a mixture:
1) of at least one non-aqueous ionic medium which is not or is only slightly miscible with the organic phase;
2) at least one compound of a catalytic element;
3) and optionally at least one co-catalyst;
in which the feed to be treated and said non-aqueous medium circulate as counter-currents between at least two treatment loops, each loop comprising at least one reaction zone connected to at least one zone for separating the organic and polar phases, the feed to be treated being supplied to the reaction zone of the first loop and fresh non-aqueous ionic medium being introduced to the reaction zone in the second or final loop, the other constituent(s) of the catalytic composition being introduced to any part of the process, and the polar phase separated in the separation zone of the second loop of each of the subsequent loops being sent to the reaction zone of the first loop or respectively the preceding loop, while the organic phase separated in the separation zone of the first loop or its subsequent loops is sent to the reaction zone of the second loop or respectively its subsequent loop, the organic phase obtained from the separation zone of the final loop and the polar phase obtained from the separation zone of the first loop being withdrawn from the process.

2. A process according to claim 1, in which a portion of the reaction medium from one reaction zone is withdrawn from one part of said zone for reinjection into said zone.

3. A process according to claim 1 or claim 2, in which at least a portion of the polar phase withdrawn from one separation zone of a loop is recycled to the reaction zone of the same loop.

4. A process according to any one of the preceding claims, in which said fresh non-aqueous ionic medium also comprises at least a portion of at least one constituent of the catalytic composition.

5. A process according to claim 4, in which the fresh non-aqueous ionic medium introduced to part of the final loop also comprises at least one transition element compound.

6. A process according to any one of the preceding claims, in which at least a portion of at least one constituent of the catalytic composition is introduced to part of the reaction zone of the first loop.

7. A process according to claim 6, in which fresh co-catalyst is introduced into the reaction zone of the first loop.

8. A process according to any one of the preceding claims, in which a fresh catalytic composition comprising fresh non-aqueous ionic medium and at least one transition element compound and optionally at least one co-catalyst are introduced into the reaction zone of the last loop.

9. A process according to any one of the preceding claims, comprising a first and a second treatment loop, each comprising a reaction zone (respectively A₁ and A₂) connected to a separation zone (respectively B₁ and B₂),
• the feed to be treated is supplied to the first reaction zone A₁ along with co-catalyst, fresh non-aqueous ionic medium mixed with at least one transition element compound and optionally at least a portion of the co-catalyst being supplied to the second reaction zone A₂;
• the polar phase separated from separation zone B₂ is introduced into the reaction zone A₁, while the organic phase separated in separation zone B₁ is introduced into reaction zone A₂;
• the organic phase containing the reaction products separated in separation zone B₂ and the used polar phase separated in separation zone B₁ being withdrawn from the process.

10. A process according to claim 2 or claim 9, in which a portion of the reaction medium is withdrawn from the reaction zone of the second loop, cooled and re-injected into that zone.

11. A process according to any one of the preceding claims, in which the non-aqueous ionic medium comprises at least one salt with formula Q⁺A⁻ where Q⁺ is a quaternary ammonium salt or a quaternary phosphonium salt and A⁻ is a co-ordinating or non co-ordinating anion.

12. A process according to claim 11, in which the anion is selected from the group formed by halogenoaluminates, organohalogenoaluminates, halogenogallates, and organohalogenogallates.

13. A process according to any one of claims 1 to 10, in which the polar phase comprises a mixture of at least one quaternary ammonium halide or quaternary phosphonium halide with at least one aluminium halide and/or at least one hydrocarbylaluminium dihalide.

14. A process according to any one of claims 1 to 10, in which the non-aqueous ionic medium results from a mixing at least one lithium halide with at least one hydrocarbylaluminium halide.

15. A process according to any one of the preceding claims, in which the polar phase comprises a mixture of at least one aluminium halide or respectively at least one hydrocarbylaluminium halide with at least one quaternary ammonium halide and/or quaternary phosphonium halide, or respectively lithium, in a molar ratio which is in the range 1 to 2.

16. A process according to any one of the preceding claims, in which the catalytic composition contains at least one catalytic element selected from the group formed by elements from groups 6, 8, 9 and 10.

17. A process according to any one of the preceding claims, used for dimerisation, co-dimerisation or oligomerisation of olefins.

18. A process according to claim 17, in which the catalytic composition comprises at least one nickel compound, at least one quaternary ammonium halide and/or quaternary phosphonium halide, and at least one aluminium halide and/or at least one hydrocarbylaluminium dihalide.

19. A process according to any one of claims 1 to 16, used for olefin metathesis.

20. A process according to claim 19, in which the catalytic element is tungsten and/or molybdenum, the co-catalyst is an organometallic aluminium compound, and the non-aqueous ionic medium results from a mixture of at least one quaternary ammonium halide and/or quaternary phosphonium halide, and at least one aluminium halide and/or at least one hydrocarbylaluminium dihalide.

21. A unit for carrying out the reaction in a non-aqueous ionic medium which is not or is only slightly miscible with olefins, comprising:
• at least two reaction zones A₁ and A₂;
• at least two separation zones B₁ and B₂ for separating the hydrocarbon phase from the polar phase, said zones being connected to the reaction zones A₁ and A₂;
• at least one conduit 1 for introducing polar phase into reaction zone A₂;
• at least one conduit 2 for introducing olefin feed into reaction zone A₁;
• at least one conduit 3 for supplying hydrocarbon phase separated in separation zone B₁ to reaction zone A₂;
• at least one conduit 4 for withdrawing hydrocarbon phase separated in separation zone B₂;
• at least one conduit 5 for sending the polar phase separated in separation zone B₂ to reaction zone A₁;
• at least one conduit 6 for withdrawing used polar phase separated in separation zone B₁.

22. A unit according to claim 21, further comprising at least one conduit 7 connecting separation zone B₂ to reaction zone A₂ to recycle the polar phase.

23. A unit according to claim 21 or claim 22, further comprising at least one conduit 8 for withdrawing a portion of the reaction medium from zone A₂ and re-introducing it to said zone.

24. A unit according to any one of claims 21 to 23, further comprising at least one conduit 9 for withdrawing a portion of the reaction medium from zone A₁ and re-introducing it to said zone.

25. A unit according to any one of claims 21 to 24, further comprising at least one conduit 10 connecting separation zone B₁ to zone A₁ for recycling the polar phase.

26. A unit according to any one of claims 21 to 25, further comprising at least one conduit 11 for introducing the reactants into the unit.

27. A unit according to any one of claims 21 to 26, in which conduit 8 and 9 comprise heat exchangers.

28. Use of a unit according to claims 21 to 27 for dimerisation, co-dimerisation or oligomerisation of olefins.

29. Use of a unit according to claims 21 to 27 for olefin metathesis.
